# FASCICULE DE BREVET EUROPEEN

(11) **EP 0 814 761 B1**
(45) Date de publication et mention de la délivrance du brevet: **18.07.2001**
(21) Numéro de dépôt: 96905910.4
(22) Date de dépôt: 05.03.1996
(51) Int. Cl.: A61K 7/48

(54) **COMPOSITION A BASE DE PLANTES POUR LE TRAITEMENT DE L'ACNE ET PROCEDE DE PREPARATION D'UNE TELLE COMPOSITION**
ZUSAMMENSETZUNG AUF PFLANZENBASIS ZUR BEHANDLUNG VON AKNE UND VERFAHREN ZUR HERSTELLUNG
PLANT-BASED COMPOSITION FOR TREATING ACNE, AND METHOD FOR PREPARING SAME

(30) Priorité: 10.03.1995 FR 9503157
(43) Date de publication de la demande: 07.01.1998
(73) Titulaire: GATTEFOSSE S.A., 69800 Saint Priest (FR)
(72) Inventeur: BURIGANA, Véronique, F-69003 Lyon (FR); ROUX, Denis, F-38360 Noyarey (FR)
(74) Mandataire: Vuillermoz, Bruno
(86) Numéro de dépôt international: FR9600340
(87) Numéro de publication internationale: WO9628138

(56) Documents cités:
- EP-A- 0 470 017
- CHEMICAL ABSTRACTS, vol. 121, no. 10, 5 Septembre 1994 Columbus, Ohio, US; abstract no. 117348, H. MATSUNAKA: "Skin-lightening cosmetics containing Santalum album extract" XP002005249 cité dans la demande & JP,A,06 048 931 (SANSEI SEIYAKU KK) 22 Février 1994
- CHEMICAL ABSTRACTS, vol. 120, no. 22, 30 Mai 1994 Columbus, Ohio, US; abstract no. 279863, Y. KOIKAWA: "Antiaging cosmetics containing plant extracts " XP002005250 cité dans la demande & JP,A,06 024 937 (NARISU COSMETIC CO. LTD) 1 Février 1994
- RESSOURCES M DICINALES DE LA FLORE FRANÇAISE, vol. 2, 1961, PARIS (FRANCE), pages 958-962, XP002005247 "Arctostaphylos Uva-ursi" cité dans la demande
- RESSOURCES M DICINALES DE LA FLORE FRANÇAISE, vol. 2, 1961, PARIS (FRANCE), pages 703-705, XP002005248 "Bergenia cordifolia" cité dans la demande

## Description

### Domaine Technique

L'invention concerne l'utilisation d'une composition à base de plantes pour l'obtention d'une composition thérapeutique destinée au traitement de l'acné ; elle se rapporte également à un procédé pour la fabrication d'une telle composition.

Comme on le sait, l'acné est une dermatose banale dont les causes sont essentiellement hormonales. On distingue essentiellement deux formes d'acné, à savoir l'acné juvénile dite également acné vulgaire et l'acné rosacée.

Ces différentes formes de dermatose sont largement évoquées dans la littérature, de sorte qu'il n'est pas utile de les décrire ici en détail.

### Techniques antérieures

Le plus généralement, le traitement de l'acné fait principalement appel à des dérivés de la vitamine A acide et au peroxyde de benzoyle. Toutefois, ces produits sont irritants et allergisants, et provoquent des inflammations et des dessèchements de la peau.

On a également proposé de traiter ces désordres par des antibiotiques. Mais il s'agit d'une thérapeutique souvent jugée lourde. Le traitement de ces désordres se fait également par des antiseptiques, tels que par exemple l'hexamidine. Toutefois, l'efficacité de ces produits est assez moyenne, car du fait de leurs propriétés essentiellement antiseptiques, ils agissent plutôt partiellement.

L'invention pallie ces inconvénients.

Elle vise l'utilisation d'une composition à base de plantes qui ne présente pas les inconvénients des solutions proposées à ce jour.

### Description sommaire de l'invention

L'utilisation de la composition à base de plantes selon l'invention pour le traitement de l'acné se caractérise en ce qu'elle contient, comme produit actif, un extrait des feuilles d'un composé choisi dans le groupe constitué par la busserole et la saxifrage de Sibérie, seule ou en mélange entre elles.

Comme on le sait :
- la busserole, dénommée dans la pharmacopée "Arcostaphylos Uva-Ursi", est un sous-arbrisseau des montagnes de France, voisin des bruyères, dénommée également "raisin d'ours", dont les feuilles sont riches en arbutine ; ce composé est bien connu comme agent dépigmentant pour le blanchiment de la peau (voir par exemple Chemical Abstracts vol. 121, n° 10, 5 septembre 1994, abrégé n° 117348) ou pour ses propriétés astringentes et antiseptiques urinaires (voir par exemple "Ressources médicinales de la flore française" pages 958-962) ;
- la saxifrage de Sibérie, dénommée également "thé de Sibérie", connue dans la pharmacopée sous le nom de "Bergenia Cordifolia", est une plante herbacée vivace cultivée dans les jardins, et dont les feuilles renferment du tannin, mais également de l'arbutine ; ces feuilles sont bien connues comme antiseptiques urinaires ou pour le traitement des blessures légères ou de l'eczéma des mains (voir par exemple "Ressources médicinales de la flore française" pages 703-705).

La busserole et la saxifrage de Sibérie sont également décrites comme agent antioxydant entrant dans la composition de produits cosmétiques antiâge (voir par exemple Chemical Abstract, vol. 120, n° 22, 30 mai 1994, abrégé n° 279863), c'est à dire pour lutter contre le vieillissement de la peau (hyperactivité de la mélanogénèse engendrant la formation de tâches de senescences).

On ne pouvait pas prévoir que les effets des feuilles de ces plantes, seules ou en mélange, pouvaient être également utiles dans le traitement de l'acné, notamment de l'acné juvénile, puisqu'il s'agit d'un domaine totalement différent (vieillesse d'un côté, jeunesse de l'autre), dans lequel l'homme du métier est dissuadé d'aller chercher la solution à son problème.

En d'autres termes, on ne pouvait pas imaginer que les feuilles de busserole efficaces pour le blanchiment de la peau, le soient pour le traitement de l'acné. En effet, la mélanogénèse, étant à l'origine de la pigmentation de la peau, est due à un processus enzymatique, tandis que l'acné juvénile est dû à un processus hormonal et bactérien.

De même, il n'était pas prévisible que les feuilles de saxifrage de Sibérie et/ou de busserole connues pour être efficaces comme antiseptique urinaire, puissent avoir également des propriétés bactéricides sur les germes spécifiques de l'acné, à savoir le Propionibactérium Acnes, le Staphylococcus Epidermidis et le Staphylococcus Aureus.

Par ailleurs, il est tout à fait suprenant que ces plantes, connues pour être efficaces comme agent antioxydant, le soient également comme bactéricide des germes spécifiques de l'acné. En effet les réactions mises en jeu sont différentes puisque les premières sont de nature enzymatique, tandis que les secondes sont de nature hormonale et bactérienne. En outre, il n'était pas évident pour l'homme du métier, cherchant à résoudre le problème de l'acné présenté par les peaux jeunes, d'aller chercher la solution dans le traitement du vieillissement de la peau concernant les peaux agées.

Dans l'application au traitement de l'acné, on peut penser que les extraits des feuilles de busserole ou de la saxifrage de Sibérie qui sont riches en arbutine, s'hydrolysent au contact de la peau pour donner de l'hydroquinone. Cette hydroquinone dont l'action est renforcée par celle contenue dans les extraits de départ, potentialise les effets bactéricides de l'arbutine sur les germes spécifiques de l'acné.

Ainsi, l'invention réside dans la sélection des extraits de feuilles de la busserole ou de la saxifrage, utilisée seule ou en mélange, comme bactéricide spécifique du traitement de l'acné.

Si on peut avantageusement utiliser de préférence les extraits des feuilles de saxifrage dont la teneur en arbutine est plus élevée, en revanche, ce composé est plus cher que la busserole, ce qui fait que l'on peut indifféremment utiliser l'un ou l'autre de ces deux produits.

Dans une forme d'exécution, on utilise comme produits actifs un mélange d'extraits à raison de moitié de busserole et de saxifrage de Sibérie.

On a observé que pour obtenir de bons résultats, la quantité en produits secs d'extrait de busserole et/ou de saxifrage dans le poids total de la composition (concentration bactéricide), doit être comprise entre 0,1 et 7 %, de préférence au voisinage de 4 %. Si la quantité introduite est inférieure à 0,1 %, on observe aucune action bactéricide sur les germes spécifiques de l'acné. En pratique, la concentration minimum bactéricide nécessaire pour l'élimination du Staphylococcus Epidermidis et du Staphylococcus Aureus est inférieure à 0,1%, tandis qu'elle est de 0,25% pour le Propionibactérium Acnes. Si cette quantité excède 7 %, on observe aucune amélioration proportionnelle.

Comme déjà dit, avantageusement, la composition doit contenir aux environs de 3 à 4 % d'extrait sec de feuilles de busserole et/ou de saxifrage.

La composition conforme à l'invention, qui peut être utilisée aussi bien en cosmétique qu'en traitement médical, peut se présenter sous des formes les plus diverses, tels que gels nettoyants ou moussants, crèmes du type eau dans huile (E/H) ou huile dans eau (H/E), masques, lotions, crèmes gels et gels.

Avantageusement, la composition est présentée sous forme de gel ou de liquide, auquel on ajoute un agent stabilisant hydrocoloïde, tel qu'une gomme de xanthane.

L'invention concerne également un procédé pour la préparation d'une telle composition pour le traitement de l'acné.

Ce procédé se caractérise en ce qu'il consiste :
- à soumettre les feuilles de busserole et/ou de saxifrage de Sibérie à une extraction hydroalcoolique ;
- à évaporer l'alcool puis à filtrer ;
- à déshydrater le filtrat obtenu ;
- et enfin, à incorporer le produit obtenu dans une composition à usage dermique pour le traitement de l'acné.

Avantageusement l'extraction hydroalcoolique est effectuée par macération.

En pratique, les feuilles de busserole et/ou de saxifrage de Sibérie sont congelées après avoir été fraichement récoltées, puis réhydratées, et enfin soumises à un traitement aux hyper-fréquences, comme décrit dans le document EP-B-0 470 017 du Demandeur.

Avantageusement, en pratique :
- de suite après avoir été cueillies, les parties aériennes (feuilles et tiges) de la plante de busserole et/ou de saxifrage de Sibérie sont congelées, puis décongelées au moment de l'utilisation par une simple immersion dans de l'eau à température ambiante avant d'être traitées aux hyper-fréquences ;
- la macération s'effectue dans un mélange d'éthanol et d'eau (à 90%), et l'évaporation sous vide de l'éthanol est effectuée à une température inférieure à 45°C ;
- la déshydratation du filtrat s'effectue par lyophilisation, voire par atomatisation.

La manière dont l'invention peut être réalisée et les avantages qui en découlent ressortiront mieux des exemples de réalisation qui suivent.

### Manières de réaliser l'invention

### Exemple 1

Au début du printemps et à la fonte des neiges, on cueille les parties aériennes (feuilles et tiges) de busserole, de manière à obtenir la concentration optimale en arbutine. Dans les quatre heures qui suivent la récolte, on congèle ces parties à moins 35°C.

Lorsqu'on désire préparer la composition conforme à l'invention, on décongèle ces parties par simple immersion dans l'eau du réseau, puis après égouttage, on place les parties réhydratées dans un four à micro-ondes pendant trente minutes environ (fréquence d'émission de 2450 MHz) pour obtenir au coeur des parties réhydratées une température voisine de 85°C. Dès qu'on obtient cette température, on arrête le four.

Dans une variante, partie essentielle seulement de la busserole est réhydratée après avoir été congelée et stabilisée aux hyper-fréquences.

On retire alors les parties traitées que l'on sèche à l'air libre et on broie. On obtient alors une poudre de couleur verte .

Cette poudre de busserole est ensuite mise à macérer dans de l'éthanol à 10° à une température de 4°C pendant quarante huit heures.

On évapore ensuite l'éthanol sous vide à une température inférieure à 45°C, et on filtre sur filtre-plaques, jusqu'à 0,22 micromètre si nécessaire.

On déshydrate ensuite le filtrat obtenu par lyophilisation dans les conditions ci-après, à savoir :
- congélation pendant deux heures à moins 40°C,
- puis, désorption sans chauffage pendant huit heures,
- enfin, chauffage progressif pendant seize heures, pour atteindre une température finale de 35°C sous un vide de 0,1 à 0,2 bar.

On obtient alors une poudre beige, hydrosoluble, présentant une faible odeur caractéristique de busserole que l'on dilue dans de l'eau distillée, pour obtenir une solution à 10 % en poids.

Séparément, de manière connue, on confectionne un gel de polymère acrylique d'usage courant pour la fabrication des cosmétiques dans lequel on introduit à température ambiante la solution à 10 % d'extrait de busserole.

On obtient alors un gel opaque que l'on applique sur la zone à traiter du visage deux fois par jour pendant deux mois environ. On observe alors la quasi-disparition des boutons d'acné.

### Exemple 2

On répète l'exemple 1 en supprimant l'étape de traitement au micro-ondes.

Au bout de trois mois de traitement, on obtient un effet similaire sur l'acné juvénile, qu'après deux mois de traitement avec un gel dont l'extrait de busserole a subi le traitement haute fréquence (exemple 1).

### Exemple 3

On répète l'exemple 1 en supprimant l'étape de déshydratation du filtrat.

On ajoute au filtrat, sous agitation rapide de l'ordre de 600 tours par minute, à température ambiante, un agent gélifiant ainsi qu'un agent stabilisant hybrocoloïde tel que de la gomme de xanthane, de manière à obtenir un gel homogène et stable.

Cette forme gel confère à l'extrait de busserole une efficacité plus rapide.

Cette forme facilite également la mise en oeuvre et l'élaboration du produit fini par l'utilisateur final. En effet, le gel ainsi obtenu peut être transformé et incorporé dans des formes finales, telles que gel, crème ou lotion.

### Exemple 4

On répète l'exemple 3 en supprimant l'étape de traitement au micro-ondes.

L'efficacité est similaire à celle obtenue dans l'exemple 3, mais est moins rapide.

### Exemple 5

On répète l'exemple 1 jusqu'à l'obtention de la poudre beige hydrosoluble.

Parallèlement, on prépare un gel nettoyant contenant pour cent parties en poids :
- 42 parties d'agent moussant ;
- 2 parties d'agent gélifiant ;
- 52,4 parties d'eau déminéralisée ;
- 0,1 partie d'un agent anti-inflammatoire (allantoïne) ;
- une partie d'un agent conservateur connu sous la marque "SEPICIDE HD" de SEPPIC;
- 0,5 partie d'un parfum ;
- 1,5 partie d'un agent solubilisant ;
- et 0,5 partie de la poudre beige hydrosoluble de feuilles de busserole de l'exemple 1.

On obtient un gel que l'on applique pour la toilette du visage à raison de deux fois par jour (le matin et le soir).

Au bout de un mois de traitement, en associant ce gel à une crème contenant la même poudre beige hydrosoluble de feuilles de busserole de l'exemple 1, on observe la quasi-disparition des boutons d'acné.

### Exemple 6

A partir de la solution mère diluée dans l'eau à 10 % en poids de poudre beige de feuilles de busserole, on réalise des dilutions sériées en tubes, dans un bouillon nutritif à raison de deux millilitres par tube. On ajoute une suspension contenant 10⁵ à 10⁶ germes par millilitre (inoculum) aux différentes dilutions de deux millilitres. On dénombre après quarante-huit heures d'incubation à 37°C les germes survivants par repiquage sur milieu gélosé.

On observe que sur les trois principaux germes de l'acné, chacun dans un milieu de culture déterminé, on obtient la concentration minimale bactéricide CMB (c'est-à-dire le nombre de germes survivants est inférieur à 0,01 % de l'inoculum), dans les conditions suivantes :
- pour le Propionibacterium Acnes sur un bouillon coeur cerveau, la CMB est de 0,25 %
- pour le Staphylococcus epidermidis sur un bouillon de Trypticase-soja, la CMB est inférieure à 0,1% ;
- pour le Staphylococcus aureus sur un bouillon de Trypticase-soja, la CMB est inférieure à 0,1%.

Dans l'interprétation de ces résultats, il faut tenir compte du fait que la solution mère, en présence de bouillons de culture, flocule, ce qui provoque une précipitation correspondant à une perte d'environ 20 % d'arbutine.

De ces résultats, on observe que la concentration optimale est bien comprise entre 1 et 4 % du poids de la composition.

L'utilisation des compositions selon l'invention concerne les formes les plus variées pour le traitement de l'acné notamment sous forme de produits nettoyants de la peau, d'après-rasage ou de crèmes traitantes.

### Exemple 7

De manière connue, on prépare une crème dermique contenant pour cent parties:
- 7 parties d'agent émulsifiant ;
- 10,5 parties d'agent émoliant ;
- 0,35 partie d'agent gélifiant ;
- 0,5 partie de conservateur ;
- 0,3 partie de parfum ;
- 0,7 partie d'une solution à dix pour cent de NaOH ;
- 1,5 partie de feuilles de saxifrage de Sibérie préparées selon les enseignements de l'exemple 1 ;
- 79,15 parties d'eau.

On obtient une crème beige que l'on applique sur la zone à traiter à raison de deux fois par jour. Au bout de deux mois de traitement, on observe la quasi disparition des boutons d'acné.

## Revendications

1. Utilisation d'une composition à base de plantes pour l'obtention d'une composition thérapeutique destinée au traitement de l'acné, caractérisée en ce que ladite composition contient, comme produit actif, un extrait des feuilles d'un composé choisi dans le groupe constitué par la busserole et la saxifrage de Sibérie, seul ou en mélange entre eux.

2. Utilisation d'une composition à base de plantes pour l'obtention d'une composition bactéricide vis-à-vis des germes Propionibactérium Acnes, Staphylococcus Epidermidis et Staphylococcus Aureus, caractérisée en ce que ladite composition contient, comme produit actif, un extrait des feuilles d'un composé choisi dans le groupe constitué par la busserole et la saxifrage de Sibérie, seul ou en mélange entre eux.

3. Utilisation non thérapeutique d'une composition à base de plantes comme agent nettoyant, caractérisée en ce que ladite composition contient, comme produit actif, un extrait des feuilles d'un composé choisi dans le groupe constitué par la busserole et la saxifrage de Sibérie, seul ou en mélange entre eux.

4. Utilisation d'une composition à base de plantes selon l'une des revendications 1 à 3, caractérisée en ce qu'elle contient, en poids de produits secs d'extrait de feuilles de busserole ou de saxifrage de Sibérie, de 0,1 à 7 % du poids total de la composition.

5. Utilisation d'une composition à base de plantes selon la revendication 4, caractérisée en ce qu'elle contient en poids de produits secs d'extrait de busserole ou de saxifrage de Sibérie, une valeur voisine de 3 à 4 % du poids total de la composition.

6. Utilisation d'une composition à base de plantes selon l'une des revendications 1 à 3, caractérisée en ce qu'elle contient comme produits actifs, un mélange d'extraits à raison de moitié de feuilles de busserole et de feuilles de saxifrage de Sibérie.

7. Procédé pour la préparation d'une composition à base de plantes utilisée selon l'une des revendications 1 à 6, caractérisé en ce qu'il consiste :
- à soumettre les feuilles de busserole et/ou de saxifrage de Sibérie à une extraction hydroalcoolique ;
- à évaporer l'alcool puis à filtrer ;
- à déshydrater le filtrat obtenu ;
- et enfin, à incorporer le produit obtenu dans une composition à usage dermique.

8. Procédé pour la préparation d'une composition à base de plantes selon la revendication 7, caractérisé en ce que l'extraction hydroalcoolique est effectuée par macération.

9. Procédé selon la revendication 8, caractérisé en ce que la macération s'effectue dans un mélange eau-éthanol et l'évaporation sous vide de l'éfhanol s'effectue à une température inférieure à 45° C.

10. Procédé selon la revendication 9, caractérisé. en ce que les feuilles fraîches, sont congelées après la cueillette, puis lors de l'utilisation, décongelées par une simple immersion dans de l'eau à température ambiante, puis soumises à un traitement aux hyper-fréquences, et enfin broyées avant d'être soumises à la macération.

11. Procédé selon la revendication 10, caractérisé en ce que la déshydratation du filtrat s'effectue par lyophilisation ou par atomisation.

## Claims

1. Use of a plant-based composition for the preparation of a therapeutic composition intended for the treatment of acne, characterized in that the said composition comprises, as active product, an extract of the leaves of a plant chosen from the group consisting of bearberry and Siberian saxifrage, alone or as a mixture thereof.

2. Use of a plant-based composition for the preparation of a composition which is bactericidal with respect to Propionibacterium acnes, Staphylococcus epidermidis and Staphylococcus aureus microorganisms, characterized in that the said composition comprises, as active product, an extract of the leaves of a plant chosen from the group consisting of bearberry and Siberian saxifrage, alone or as a mixture thereof.

3. Non-therapeutic use of a plant-based composition as cleansing agent, characterized in that the said composition comprises, as active product, an extract of the leaves of a plant chosen from the group consisting of bearberry and Siberian saxifrage, alone or as a mixture thereof.

4. Use of a plant-based composition according to one of Claims 1 to 3, characterized in that it comprises, as weight of dry products, from 0.1 to 7% of the total weight of the composition of extract of bearberry or Siberian saxifrage leaves.

5. Use of a plant-based composition according to Claim 4, characterized in that it comprises, as weight of dry products, a value of approximately 3 to 4% of the total weight of the composition of extract of bearberry or Siberian saxifrage.

6. Use of a plant-based composition according to one of Claims 1 to 3, characterized in that it comprises, as active products, a mixture of extracts in the proportion of half of bearberry leaves and of Siberian saxifrage leaves.

7. Process for the preparation of a plant-based composition used according to one of Claims 1 to 6, characterized in that it consists:
- in subjecting bearberry and/or Siberian saxifrage leaves to aqueous/alcoholic extraction;
- in evaporating the alcohol and in then filtering;
- in removing water from the filtrate obtained;
- and, finally, in incorporating the product obtained in a composition for dermal use.

8. Process for the preparation of a plant-based composition according to Claim 7, characterized in that the aqueous/alcoholic extraction is carried out by maceration.

9. Process according to Claim 8, characterized in that the maceration is carried out in a water/ethanol mixture and the evaporation under vacuum of the ethanol is carried out at a temperature of less than 45°C.

10. Process according to Claim 9, characterized in that the fresh leaves are frozen after picking, then, during use, defrosted by simple immersion in water at ambient temperature, then subjected to treatment with ultrahigh frequency radiation and, finally, milled before being subjected to maceration.

11. Process according to Claim 10, characterized in that the removal of water from the filtrate is carried out by lyophilization or by atomization.

## Patentansprüche

1. Verwendung einer Zusammensetzung auf Pflanzenbasis zur Gewinnung einer zur Behandlung von Akne bestimmten therapeutischen Zusammensetzung, **dadurch gekennzeichnet**, daß die genannte Zusammensetzung als Wirkstoff ein Extrakt aus Blättern einer Mischung enthält, welche aus der Gruppe bestehend aus der Bärentraube und der Bergenie, allein oder miteinander vermischt, ausgewählt wird.

2. Verwendung einer Zusammensetzung auf Pflanzenbasis zur Gewinnung einer bakteriziden Zusammensetzung gegen die Keime Propionibacterium Acnes, Staphylococcus Epidermidis und Staphylococcus Aureus, **dadurch gekennzeichnet**, daß die genannte Zusammensetzung als Wirkstoff ein Extrakt aus Blättern einer Mischung enthält, welche aus der Gruppe bestehend aus der Bärentraube und der Bergenie, allein oder miteinander vermischt, ausgewählt wird.

3. Nicht-therapeutische Verwendung einer Zusammensetzung auf Pflanzenbasis als Reinigungsmittel, **dadurch gekennzeichnet**, daß die genannte Zusammensetzung als Wirkstoff ein Extrakt aus Blättern einer Mischung enthält, welche aus der Gruppe bestehend aus der Bärentraube und der Bergenie, allein oder miteinander vermischt, ausgewählt wird.

4. Verwendung einer Zusammensetzung auf Pflanzenbasis nach einem der Ansprüche 1 bis 3, **dadurch gekennzeichnet**, daß der Gewichtsanteil an Trockenstoffen des Extrakts aus Blättern der Bärentraube oder der Bergenie zwischen 0,1 und 7 % des Gesamtgewichts der Zusammensetzung ausmacht.

5. Verwendung einer Zusammensetzung auf Pflanzenbasis nach Anspruch 4, **dadurch gekennzeichnet**, daß der Gewichtsanteil an Trockenstoffen des Extrakts aus Blättern der Bärentraube oder der Bergenie einen Wert nahe 3 bis 4 % des Gesamtgewichts der Zusammensetzung ausmacht.

6. Verwendung einer Zusammensetzung auf Pflanzenbasis nach einem der Ansprüche 1 bis 3, **dadurch gekennzeichnet**, daß sie als Wirkstoff eine aus jeweils zur Hälfte aus Extrakten aus Blättern der Bärentraube und Extrakten aus Blättern der Bergenie bestehende Mischung enthält.

7. Verfahren zur Darstellung einer gemäß einem der Ansprüche 1 bis 6 verwendeten Zusammensetzung auf Pflanzenbasis, **dadurch gekennzeichnet,** daß es darin besteht:
- die Bärentrauben- und/oder Bergenienblätter einer hydroalkoholischen Extraktion zu unterziehen;
- den Alkohol zu verdampfen und den so erhaltenen Stoff zu filtrieren;
- das erhaltene Filtrat zu dehydratisieren;
- und schließlich den erhaltenen Stoff einer Zusammensetzung zur Anwendung auf der Haut beizumengen.

8. Verfahren zur Darstellung einer Zusammensetzung auf Pflanzenbasis nach Anspruch 7, **dadurch gekennzeichnet**, daß die hydroalkoholische Extraktion durch Mazeration erfolgt.

9. Verfahren nach Anspruch 8, **dadurch gekennzeichnet**, daß die Mazeration in einem Wasser-Ethanol-Gemisch erfolgt und das Verdampfen unter Vakuum des Ethanols bei einer Temperatur unter 45° C erfolgt.

10. Verfahren nach Anspruch 9, **dadurch gekennzeichnet,** daß die frischen Blätter nach dem Pflücken tiefgefroren und dann bei Verwendung durch einfaches Eintauchen in Wasser bei Umgebungstemperatur aufgetaut werden, danach einer Ultrahochfrequenzbehandlung unterzogen werden und schließlich gemahlen werden, bevor sie der Mazeration unterzogen werden.

11. Verfahren nach Anspruch 10, **dadurch gekennzeichnet**, daß die Dehydratation des Filtrats durch Gefriertrocknen oder durch Zerstäuben erfolgt.
